# EUROPEAN PATENT APPLICATION

(11) **EP 1 338 294 A2**
(43) Date of publication of application: **27.08.2003**
(21) Application number: 03003237.9
(22) Date of filing: 21.02.2003
(51) Int. Cl.: A61M 1/10

(54) **Method and apparatus for providing limited back-flow in a blood pump during a non-pumping state**

(30) Priority: 21.02.2002 US 358550 P; 19.12.2002 US 325275
(71) Applicant: VASCOR, Inc., Pittsburgh, PA 15238-2960 (US); UNIVERSITY OF PITTSBURGH, Pittsburgh, Pennsylvania 15260 (US)
(72) Inventor: Heilman, Marlin S., Sarver, Pennsylvania 16055 (US); Capone, Christopher D., Pittsburgh, Pennsylvania 15238 (US); Kolenik, Steve A., Leechburg, Pennsylvania 15656 (US); Moore, Daniel R., Allison Park, Pennsylvania 15101 (US); Parisi, Carl M., R.D., Pennsylvania 16201 (US); Prem, Edward K., Allison Park, Pennsylvania 15101 (US); Sofranko; Richard A., Pittsburgh, Pennsylvania 15227 (US); Antaki, James, Allison Park, Pennsylvania 15101 (US); Paden, Brad, Santa Barbara, California 93111 (US); Borzelleca, David, Wexford, Pennsylvania 15090 (US); Burgreen, Greg, Bakerstown, Pennsylvania 15007 (US); Holmes, John A., Wexford, Pennsylvania 15090 (US); Wu, Zhongjun, Wexford, Pennsylvania 15090 (US)
(74) Representative: Banzer, Hans-Jörg, Dipl.-Ing.

(57) **Abstract**

A method and apparatus as described herein for substantially, but not entirely, blocking back-flow through a blood pump when the blood pump is not pumping blood wherein the method and apparatus can operate passively or actively if the blood pump stops pumping in order to provide a limited back-flow through the blood pump to prevent clot formation.

## Description

### BACKGROUND

Serious heart failure, or the inability of a person's heart to pump sufficient blood for their body's needs, is the cause of very poor quality of life, huge medical treatment costs, and death in hundreds of thousands of patients yearly. Each year, thousands of patients in end-stage heart failure need circulatory assist devices as a life saving measure. These devices are primarily left ventricular assist devices, which, unlike a total artificial heart, leave the native heart intact and provide a pressure boost to the blood delivered from the patient's heart.

A left ventricular assist device typically has an inflow conduit attached to the left ventricle and an outflow conduit connected to the aorta. This connection scheme places the pump in parallel with the native left ventricle and allows the pump to assist the patient's circulation by supplying pressurized blood to the aorta. The parallel connection also allows the heart to pump blood directly into the aorta whether the pump is operating or not. This provides a safety margin for the patient, since a pump failure wouldn't necessarily result in death if the patient's heart were still capable of pumping sufficient blood to maintain life. However, depending on the type of pump used or whether other flow modifying devices, such as valves are present, the patient may still be at great risk from pump failure. Typically, parallel pulsatile pumps have heart valves within the flow path so that blood can only move in a forward direction from the heart to the aorta through the parallel path. If a pulsatile pump fails, the blood within the parallel path usually becomes totally stagnant. The valves beneficially prevent back-flow from the aorta to the left ventricle that would defeat the pumping action of the heart, but the valves can present the serious problem of blood stagnation and clotting in the parallel path. In minutes, the stagnant pooled blood can clot and prevent any possible reestablishment of pump operation due to the risk of introducing clots into the patient's circulation.

For continuous flow blood pumps, such valves are not typically used. Consequently, when a continuous flow pump stops, blood may flow in a reverse direction through the parallel path, resisted only by the flow impedance of the inactive pump. The pooling of blood in the pump is prevented but at the cost of excessive back-flow through the parallel blood path which defeats the pumping action of the left ventricle.

Blood pumps have been disclosed which provide for blockage of reverse flow with pump failure in continuous flow pumps. For example, the blood pump described in United States Patent No. 4,688,998 has a blood pump rotor that acts as a valve by shifting position within the blood pump housing to block reverse blood flow if the pump fails. Check valves are also known to be included as part of a blood pumping system, but externally and not associated with the blood pump, such as described in United States Patent No. 5,613,935, wherein a check valve is provided in the graft attached to the pump outlet. However, in both cases, the purpose is to completely prevent the reverse flow of blood thru the pump. In that situation, the pump cannot be restarted if left off for longer that a brief period due to the blood clotting issues mentioned above.

An additional consideration is that, during implantation, undesirable bleeding, i.e., blood flow, can occur in the reverse direction through the blood pump before the blood pump can be activated. Thus, it would also be advantageous to substantially lessen this unwanted bleeding during implantation of the blood pump.

Consequently, it can be desirable to generally restrict, yet permit a limited amount of back-flow through the blood pump when the blood pump is not operational. The small back-flow can beneficially "wash" the blood contacting surfaces and reduce the likelihood of clot formation. Yet, this reverse blood flow can be restricted sufficiently so as not to cause the type of problems that would result from a wholly unrestricted back-flow.

Provision of a limited back-flow in a blood pump, just sufficient to wash the blood contacting surfaces, can thereby address safety requirements both from the standpoint of the need to generally restrict back-flow in case of pump failure, or during implantation, and also from the standpoint of the need to prevent clot formation. Moreover, allowing a restricted back-flow can also enable a safe "pump off" mode. For example, during sedentary periods including sleep, the blood pump could be potentially safely shut down, thereby lengthening the battery life of the blood pump.

Accordingly, there is a need for a blood pump configured to substantially block back-flow through the pump in the event of pump failure, but which also permits a limited amount of back-flow through the pump for washing the blood flow path to prevent clot formation.

### SUMMARY

According to the present invention, the above object is achieved by a blood pump as defined in independent claim 1 and independent claim 2, respectively. The dependent claims define preferred or advantageous embodiments of the invention.

A blood pump having one or more channels for the passage of blood can include a valve member for substantially blocking retrograde flow of blood when the blood pump is not operational. Generally, the valve member acts as a flow-limiting valve. The valve member, in one exemplary embodiment, can be an inflatable balloon disposed generally in the center of the blood pump, and can be well suited for active control. through manipulation of a liquid or gas that is used to fill the balloon to an inflated state. In an expanded state, the balloon nearly blocks the passage of blood through the blood pump, but a small level of reverse flow is permitted to allow for washing of the pump and valve surfaces. The balloon can be made of a polymer and have a separate inner structure which prevents the balloon from completely collapsing. In an expanded state, the balloon nearly blocks the passage of blood through the blood pump, with a small level of reverse flow being permitted to allow for washing of the pump and valve surfaces.

In another embodiment the valve member can include a valve portion or portions that rotate with back-flow to partially block the passage of blood through the blood pump. For example, a single disk shaped portion can be used, or, alternatively, four separate "flappers" can be used. The valve members can change state passively as a result of a changing pressure difference across the valve member.

Other embodiments can also act passively with respect to the pressure across the valve member. For example, a continuous flexing spiral member can be used as the primary portion of the valve member. In one case, the spiral member can be substantially flat when closed and opens by stretching in an axial direction. In another case, spiral member can have a conical shape when closed and changes to an open state by stretching in an axial direction similarly to the flat spiral member.

In another embodiment, the valve member can be a dual flexing member arrangement. For example, two adjacent valve portions can lay within the central bore of the blood pump and passively flex as a function of the pressure differential across the pump. The adjacent valve portions can be designed to substantially block back-flow during periods that the blood pump is off, but to allow sufficient leakage to wash the blood pump and valve portions.

Another embodiment can be especially useful for the secondary gap of a dual gap blood pump, or for a blood pump having a single annular blood pathway. In this case, a circumferential membrane can be positioned lying across the surface of the rotor, or the pump housing. The membrane can move circumferentially into the blood pathway to achieve a partial blockage. The intrusion into the annular blood pathway may be accomplished by different methods, some passive, which rely on rotor rotational speed and others that are actively controlled. If used with a dual flow blood pump, this embodiment could also be used in conjunction with another of the previous embodiments such that both blood flow pathways can be partially occluded.

In a further embodiment, the rotor itself can be moved axially to block the blood flow path. In this case, the inlet or outlet of the blood flow path and the sealing end of the rotor can be configured such that a complete seal is not achieved. Instead, provisions can be made in the mating surfaces to permit a restricted reverse blood flow.

Other details, objects, and advantages of the invention will become apparent from the following detailed description and the accompanying drawings figures of certain embodiments thereof.

### BRIEF DESCRIPTION OF THE DRAWING FIGURES

A more complete understanding of the invention can be obtained by considering the following detailed description in conjunction with the accompanying drawings, in which:
Figure 1 is an isometric view of an embodiment of a balloon valve within a blood pump.
Figure 2 is a cross-sectional view of the balloon valve.
Figure 3a is a view of the balloon valve mounted to the blood pump volute.
Figure 3b is a view of the balloon valve mounted to the blood pump inlet.
Figures 4a is a perspective view of an embodiment of a balloon valve membrane in an uninflated state.
Figures 4b-4c show are perspective views of embodiments of an inner support frame for the balloon valve.
Figure 5a is a view of an embodiment of a disk valve in a closed state.
Figure 5b is a view of the disk valve in an open state.
Figure 6 is a perspective view of an embodiment of a flapper valve.
Figure 7 is a side view of the flapper valve with a central strut.
Figures 8a and 8b are perspective views of an embodiment of a spiral valve.
Figure 9 is a view of the spiral valve with a support structure.
Figure 10 is a perspective view of another embodiment of a spiral valve.
Figure 11 is a perspective view of an embodiment of a dual member valve.
Figure 12 is a side view of the dual member valve.
Figures 13a and 13b are projected views of the dual valve member.
Figures 14a and 14b illustrate an embodiment of a circumferential valve member.
Figures 15-17 illustrate another embodiment of a circumferential valve.
Figures 18a and 18b illustrate an additional of a circumferential valve.
Figures 19 and 20 show a further embodiment of a circumferential valve.
Figures 21 and 22 illustrate a blood pump employing an axially movable rotor as an integral back flow limiting valve.

### DETAILED DESCRIPTION

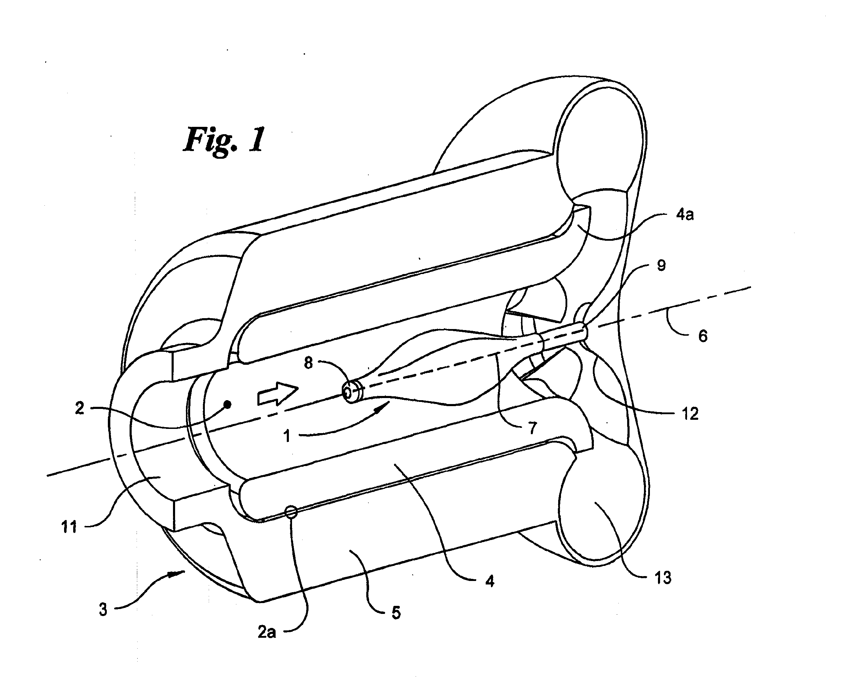

A first embodiment of the invention is depicted in Figure 1, wherein an inflatable balloon 1 is situated within the central bore 2 of an implantable blood pump 3 having a rotor 4 suspended within a stator 5. The balloon can have two operational states; the first being when completely deflated. In this state, the balloon is at its smallest volume, such that minimal impedance to forward flow is created. This state can be maintained, while the blood pump provides assist to the patient. If the blood pump is turned off for therapeutic reasons or if there is a pump failure, the balloon can be inflated to a larger volume which partially blocks the central bore of the blood pump 3. As a result, the retrograde flow through the pump 3 is reduced to a level that will not harm the patient but not completely blocked so as to keep the blood contacting surfaces washed.

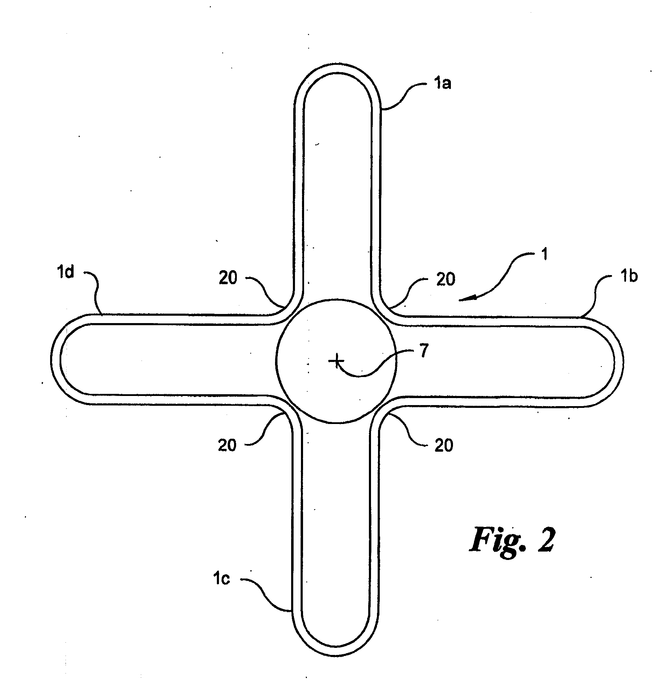

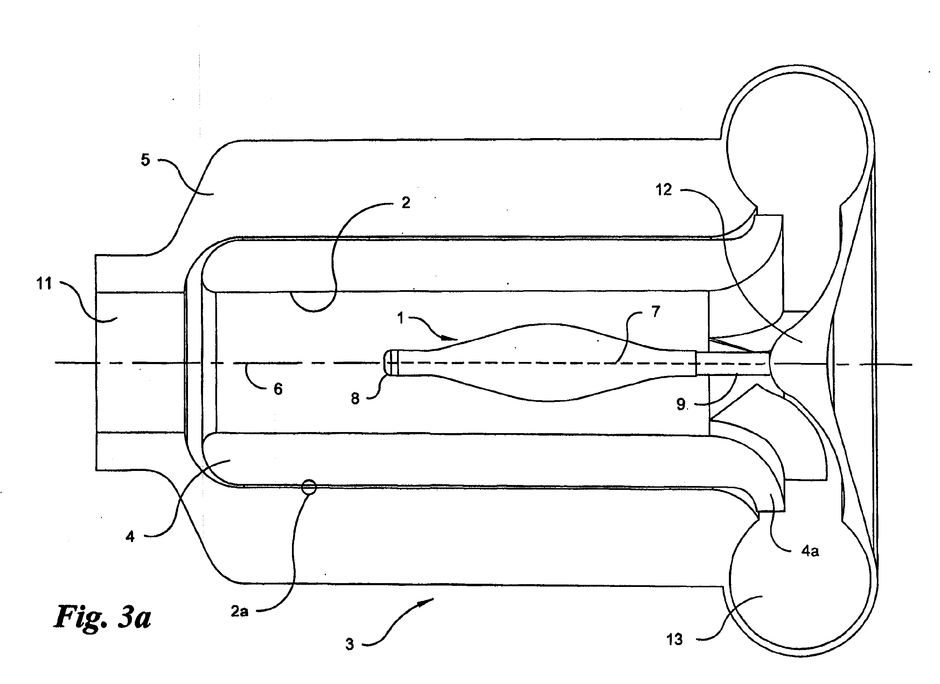

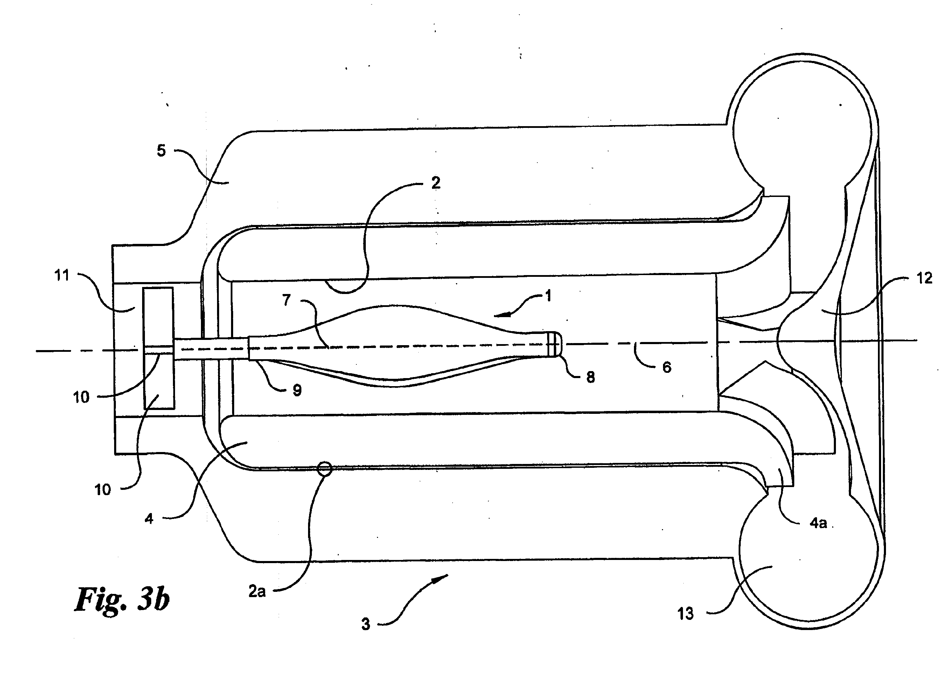

The balloon 1 can be elliptical shaped, with the long axis 7 aligned with the axis or rotation 6 of the rotor 4, such that in any state of inflation or deflation, the balloon 1 will remains generally concentric with the rotor 4. The cross-section of the balloon 1 can vary along the length of the long axis 7 of the balloon 1. In the deflated state, the balloon 1 can have the four-lobed cross-sectional shape depicted in Figure 2. However, other configurations are also possible using less or more lobes 1a-1d, depending on the needs of the invention. Along the length of the balloon 1, the cross-section can be designed to be largest at a centerpoint of the balloon length, and decreases in area as the point of view approaches either end. The largest cross-section can be at the middle of the balloon 1 as measured along the long axis 7, but can be located at different locations as desired. The balloon 1 can have a free end 8 and a fixed end 9, as depicted in Figure 3a. The fixed end 9 may either be rigidly mounted to the stator 5, such as at the volute housing 12, near the outlet 13, as shown in Figure 3a. Alternatively, the fixed end 9 can be mounted to one or more cross members 10, which can be mounted to the stator 5 near the inlet 11 of the blood pump 3, as shown in Figure 3b. Since the cross members 10 are in the blood flow path 2, they can be used to affect flow characteristics of the blood flow. For example, if the cross members 10, which can be thin, planar members, are generally aligned parallel with the blood flow, i.e., the thin edge aimed along the flow path, the cross members 10 can act as flow straighteners. If positioned at an angle to the flow path, the cross members 10 can create swirling. The geometry, either positioning or shape of the cross members 10, can be varied to produce various flow characteristics that may be advantageous.

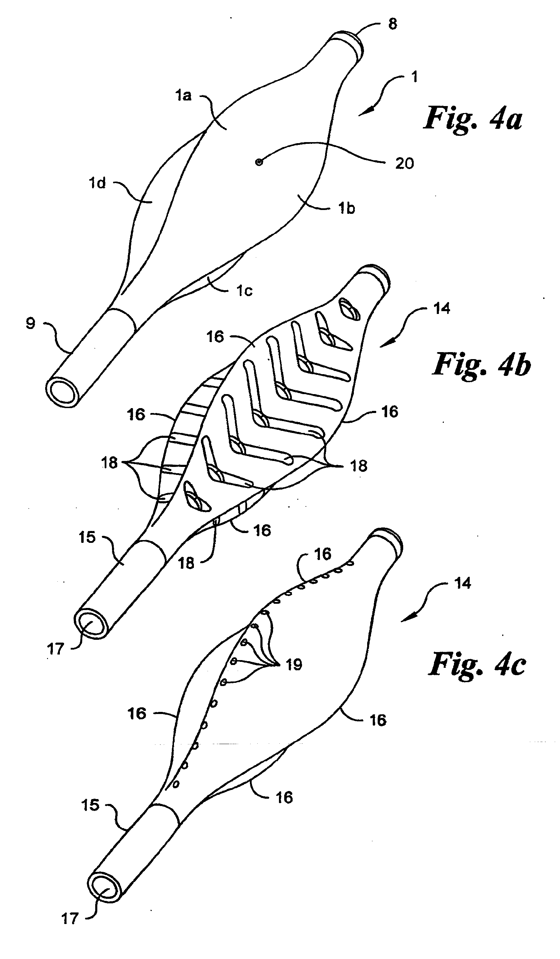

A support frame 14 can be provided for the balloon member 1, as shown in Figures 4b and 4c. The support frame 14 can have a central strut 15 and curved lobe members 16, which can support corresponding curved lobe portions 1a-1d of the uninflated balloon member 1, shown in Figure 4a. The curved members 16 can serve to add structure to the balloon 1 in the deflated state such that generally no flow or pressure condition would cause the balloon 1 to collapse upon itself. The curved members 16 may each be thin, curved, and shaped to match the form of the uninflated balloon 1 as shown in Figure 4a. The central strut 15 can have a central channel or passageway 17 that allows the passage of a liquid or gas for pressurization of the balloon 1.

Each curved member 16 can be mounted to the central strut 15 extending from the cross members 10 and can have an opposite end which terminates together with the other curved members 16. So configured, the curved members 16 form a hoop-like structure that contacts generally the outer edges of the uninflated balloon 1. For instances in which the blood pump 3 will be run in a demand mode, repeated inflations and deflations of the balloon 1 would occur for the duration of the therapy. During this period, repeated contact between the balloon 1 and the curved members 16 occurs, which can increase the likelihood of an abrasion, induced perforation of the balloon membrane. The minimal contact provided by the hoop-like structure minimizes the contact area between the balloon 1 and curved members 16 such that the chance of an abrasion-induced perforation is greatly reduced. The hoop-like structure can also be made of a biocompatible polymer and have a surface roughness which minimizes damage to the membrane of the balloon 1 by abrasion.

The hoop-like structure can also have greater rigidity. As opposed to being simple hoops, the curved members 16 can have a uniform thickness from the outer edge to the central strut 15. The frame member, and/or curved members 16 can have channels 18, or holes 19, across the surface thereof for delivery of the medium, which pressurizes the inner wall of the balloon 1 to inflate it.

The balloon 1 may have a variable number of lobes 1a-1d, as shown in Figure 2. In one embodiment, there are four lobes 1a-1d equally spaced in a circumferential manner. Each lobe 1a-1d can extend outward a distance which substantially, but not entirely, occludes the bore 2, i.e., blood flow path, of the blood pump 3. The region 20 of the balloon 1 in the deflated state, which lies close to the central strut 15, is moved radially outward with respect to the axis or rotation 7 of the rotor 4 when the balloon 1 is inflated. The balloon 1 can be designed such that when inflated, the cross-section has a constant radius, with respect to the axis or rotation 6 of the rotor 4. This radius can be sized to nearly equal the radius of the bore 2 of the blood pump 3. The clearance between the balloon 1 and central bore 2 can be designed, for example, such that approximately 50 milliliters/minute of blood can leak back through the central bore 2 during periods when the blood pump 3 is not operating, or is operating below a certain speed.

The balloon 1 can have two geometric states: fully inflated and fully deflated. Of course, various intermediate stages of inflation are also possible. The balloon 1 can be formed in the fully deflated state, and can be designed such that there is generally no stretching of the balloon 1 membrane at the fully inflated stage. Stretching of the balloon 1 membrane at the inflated state can be avoided since close control of the final, fully inflated diameter of the balloon 1 can be desirable. Since the clearance between the balloon 1 and central bore 2 can govern the magnitude of reverse flow passing through the central bore 2, additional sensors and control may need to be employed to govern the balloon 1 inflation pressure or inflated size. However, this would add complexity to the operation of the blood pump 3. The balloon 1 can be made of a biocompatible polymer that has long-term stability for permanently implanted devices.

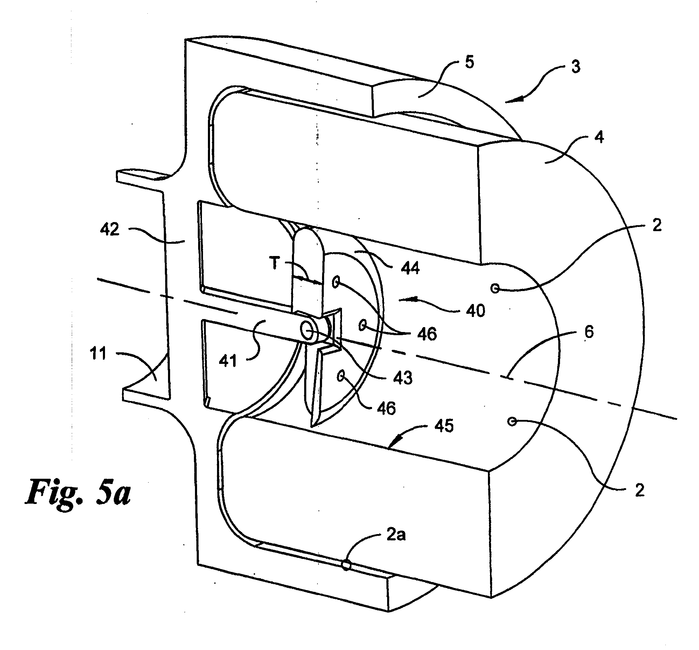

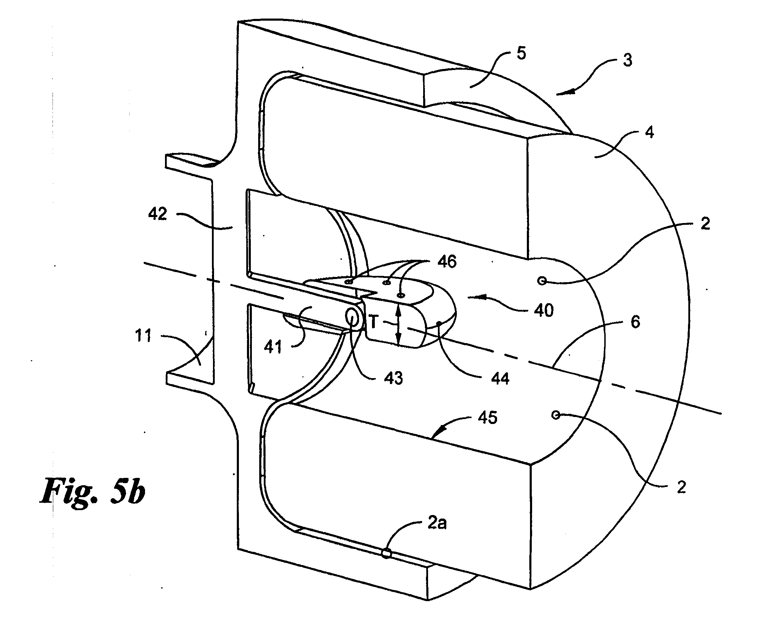

A second embodiment of the invention is depicted in Figure 5a, wherein the valve member, shown in a closed state, can be a single disk shaped valve 40 positioned within the central bore 2 of the blood pump 3. During normal blood pump 3 operation, the valve 40 can remain open, as shown in Figure 5b, such that blood entering the impeller 4a of the blood pump 3 is unimpeded. When the blood pump 3 is not operating, or the impeller 4a is rotated lower than a certain speed, the valve 40 position can change to the closed position so that the central bore 2 of the blood pump 3 can be blocked to the extent that only a limited level of backward flow is permitted. The valve 40 can be mounted on a strut 41 that can extend from a cross member 42 positioned near the inlet 11 of the blood pump 3. Multiple cross members could also be used. Additionally, the strut 41 could be attached to the volute housing 12 near the pump 3 outlet 13, similarly to the balloon valve 1 attachment illustrated in Figure 1.

A pivot 43 can be provided between the valve 40 and the support strut 41 about which the valve 40 can rotate with respect to the support strut 41. The valve 40 can be mounted off center to the support strut 41, such that the valve 40 has a tendency to remain shut when the blood pump 3 is not operational. The valve 40 generally remains shut when the pressure differential across the valve 40 is insufficient to rotate the mass of the valve 40 to an open position. During normal blood pump 3 operation, the pressure differential can be high enough to rotate the valve 40 under typical operating conditions of the blood pump 3.

A small clearance can exist between a periphery 44 of the valve 40 and the wall 45 of the central bore 2. The clearance can be uniform around the periphery 44, or it can be strategically located at various positions around the valve periphery 44. The clearance serves the purpose of allowing a small amount of reverse blood flow during periods when the blood pump 3 is off or operating at less than a certain speed. The size of the clearance can be a factor in determining the magnitude of back-flow for any given hemodynamic state of the patient. However, other passages, such as holes 46, could also be provided through the face of the valve 40 to provide limited reverse flow. The positioning of the clearance around the periphery 44, whether it be evenly distributed circumferentially, can focused in certain regions, or passages in other regions of the valve 40 body, can also be used to aid in the washing of the valve 40 surface during periods the blood pump 3 is off. The surfaces of the valve 40 may also have other features, such as raised portions, grooves, notches, etc., which can also have positive effects on the flow of blood past the valve body. These features, in general, serve to eliminate areas of stagnation near to or attached to the surface of the valve 40, the support strut 41, or the pivot joint 43 formed between the two. It should be noted that these features can produce beneficial effects regardless of the valve 40 being in an open or closed state.

During normal operation, the valve 40 can be rotated such that the thickness of the valve 40 is substantially aimed along the blood flow pathway. In this position, the valve 40 presents the minimum obstruction to forward blood flow. As the blood pump 3 rotor 4 spins and the impeller 4a pumps blood, the valve 40 remains stationary in the open position shown in Figure 5b. During this period, the valve 40 can have the added effect of straightening the blood flow entering the impeller stage. The degree of flow straightening produced can somewhat depend on the thickness profile of the valve 40 when in the open position. Also, the position of the valve 40 with respect to the impeller 4a can be varied to adjust the degree of flow straightening. For example, positioning the valve 40 closer to the volute housing 12 can substantially restrict swirling of the blood near the entrance of the blood pump 3 impeller 4a. Conversely, moving the valve 40 more toward the blood pump 3 inlet 11 can minimize the flow straightening effect the valve 40 has on the blood entering the impeller 4a.

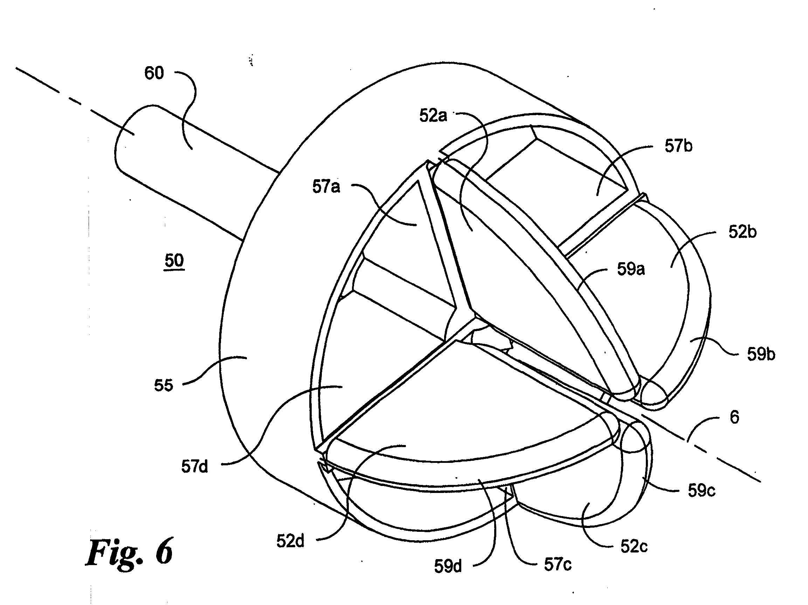

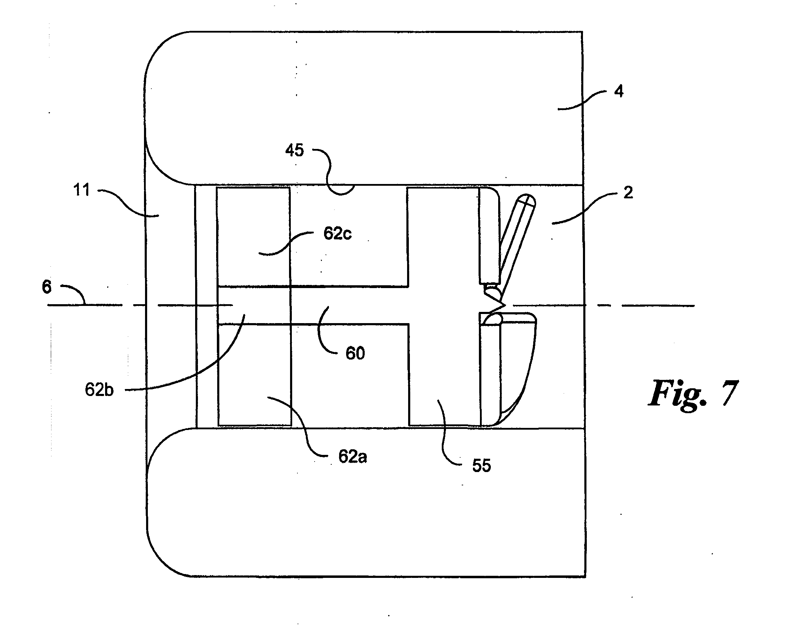

Another embodiment of the invention, a "flapper" valve 50, is depicted in Figures 6 and 7, wherein separate leaflets, in this example four leaflets 52a-52d, can be spaced around a support member 55, which can be generally cylindrical. The leaflets 52a-52d can be biased to remain shut, such that at low differential pressures across the valve 50 the leaflets 52a-52d will close and substantially block the back-flow of blood across the valve 50. By varying the geometry of the leaflets 52a-52d and the cylindrical support member 55, a desired level of which will not have a dramatic effect on the native ventricle's ability to continue pumping blood when the blood pump 3 is off. If the cylindrical support member 55 is used, the space between the outer surface of the support member 55 and the wall 45 of the central bore 2 can determine the level of back-flow permitted during periods of non operation for the blood pump 3. If the generally cylindrical support member 55 is not used, the space formed between an outer periphery 59a-59d of the leaflets 52a-52d and the wall 45 of the central bore 2 can determine the level of back-flow.

The leaflets 52a-52d of the valve 50 can be mounted to support members 57a-57d that in turn can be mounted to the cylindrical support member 55. Although described as separate, the support members 57a-57d could simply be a pair of cross members, with two leaflets mounted at opposite ends of each one. On the end of the support member 55 opposite the leaflets 52a-52d, a central strut 60 can be provided which extends away from the leaflets 52a-52d and along the axis of rotation 6 of the rotor 4. The central strut 60 could be used to position the valve 50 within the central bore 2 of the blood pump 3, such as by mounting the other end of the strut 60 to additional cross members 62a, 62b that in turn can be affixed to the bore 2 of the blood pump 3 near the inlet 11, as shown in Figure 7. Alternatively, the other end of the strut 60 could be mounted to the volute housing 12 near the impeller 4a, similarly to the mounting of the balloon valve member 1 shown in Figure 3a. By varying the length of the central strut 84, the valve 50 can be located at different positions along the central bore 2. As stated previously, there can be situations in which the positioning of the valve 50 can be more advantageous near the impeller 4a, or others in which the distance between the impeller 4a and valve 50 needs to be maximized. This is important since the flow patterns of the blood entering the impeller 4a of the blood pump 3 may, depending on the impeller 4a design, need to be manipulated to improve the function of the impeller 4a, reduce blood damage, or reduce the possibility of cavitation.

In the embodiment shown, four leaflets 52a-52d are illustrated although more or fewer leaflets 52a-52d may be used. Each leaflet 52a-52d can be mounted via the support members 57a-57d that extend from the center of the valve 50 to the generally cylindrical support member 55. Each leaflet 52a-52d can assume a position substantially aligned with the blood flow trajectory during periods of normal blood pump 3 operation. In that position, the leaflets 52a-52d can have a thickness that obstructs the flow of blood to a minimum degree. When the blood pump 3 is not operational, or operating at less than a certain speed, the pressure difference across the valve 50 can move the valve leaflets 52a-52d to a closed position wherein only a limited reverse flow is permitted, and maintained.

The leaflets 52a-52d can be made of, for example, a rigid implantable metal such as Titanium or one of its alloys. When such a metal is used, a biocompatible coating such as a polymer can cover the blood contacting surfaces, if desired. Other materials can be used for the leaflets 52a-52d, if the material strength is sufficient and if the material is implantable.

To facilitate the movement of the leaflets 52a-52d, a hinge joint may be used at the junction between each leaflet 52a-52d and corresponding support member 55 of the cylindrical support member 55. The joint allows free rotation of each leaflet 52a-52d from the closed position to the open position. If needed, the joint may also limit rotation to provide precise positioning of the leaflets 52a-52d at either extreme position. This feature can enable the positioning of the leaflets 52a-52d to produce different flow conditions around the leaflets 52a-52d and downstream of the leaflets 52a-52d. The leaflets 52a-52d may also have features such as grooves, notches, or channels that aid in washing the surface of the leaflets 52a-52d, joints, or the support members 55. The shape of the leaflet 52a-52d cross section can also be varied to produce improved washing.

The leaflets 52a-52d can be made of a flexible material like Nitinol,.which is an alloy known for the ability to flex without structural failure, and for the ability to change properties depending on the presence of electrical current applied to its structure. The use of such a material can allow for active control of leaflet 52a-52d position, and can eliminate the need for a joint at the leaflet-to-support member junction. Whereas other embodiments whose closure state changes passively as a function of pressure, this embodiment can allow greater control of the valve leaflet 52a-52d position. The Nitinol can also provide a smooth surface across which blood can flow more evenly, unlike the situation present where a hinge joint is used. Allowing the Nitinol to provide the bending action can also reduce the possibility of flow stagnation near a hinge joint.

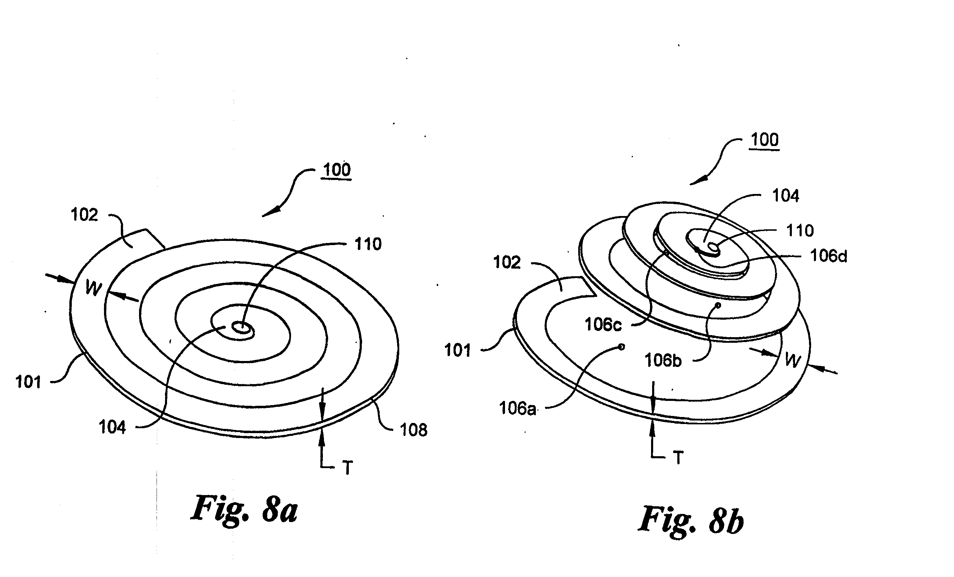

Another embodiment of the invention is depicted in Figures 8a and 8b, wherein the valve member 100 comprises a continuous flexing member 101 present within the central bore 2 of the blood pump 3. The flexible member 101 can have a spiral shape with one fixed end 102 and one free end 104 terminating near the center of the spiral. When collapsed, the flexible member 101 can be substantially flat, with the free end 104 in generally the same plane as the fixed end 102, as shown in Figure 8a. In this collapsed position, which corresponds to a non-pumping state, the diameter of the spiral is nearly the same as the diameter of the blood flow path and thus can substantially block the back-flow of blood. As shown in Figure 8b, when the blood pressure exceeds a predetermined level, the free end of the flexible member 101 is designed to translate along the axis of rotation 6 of the rotor 4 in the direction of the flow of blood, thereby forming a generally conical shape, wherein spaces, such as spaces 106a-106d, form between adjacent edges of the spiral to permit forward blood flow with less impedance. In a non-pumping condition, the flexing member 101 collapses back to the generally flat shape. In this position, only a limited back-flow of blood is permitted, such as through a narrow clearance provided between the periphery 108 of the spiral and the bore 2 of the blood pump 3, which provides washing of the surface of the valve 100. As in previous embodiments, placement of the valve 100 within the central bore 2 can vary depending on the level of interaction with the impeller 4a that is sought. Closer placement to the impeller 4a can have a greater effect than distant placement.

The behavior of the valve 100 can be dictated by its structural characteristics. The material can be a biocompatible alloy, like Nitinol, which is capable of large deflections and strains without approaching stress levels that could otherwise cause failure of the flexible member 101. The flexible member 101 can have a substantially uniform width "W" along the spiraling length. However, varying the width along the spiral can also be utilized to affect the flexing characteristics of the flexible member 101. For a given blood pump 3 central bore 2 diameter, varying the width W of the flexible member 102 can result in a change in the number of spiral wraps. Additionally, the width W of the flexible member 101 can also be varied as a function of angular position with respect to the center of the valve 100. Similarly, the thickness "T" along the length of the flexible member 102 can be uniform along the length of the spiral. Alternatively, the thickness T can be varied to control the deflection behavior of the flexible member 101. As an example, for a flexible member 101 of uniform width W and thickness T, the flexible member 101 will tend to have the largest deflection at the greatest diameter and, measuring length along the spiral, the deflection will decrease as the center of the valve 100 is approached. If the center of the valve 100 is desired to deflect to a greater degree, then the width W and/or thickness T of the flexible member 101 can be varied to change the deflecting behavior of the valve 100.

During a blood pump 3 off period, the closed valve 100 can preferably be washed by a limited back flow around the periphery of the spiral 108, through the clearance between the periphery and the central bore 2 of the blood pump 3. The valve 100 can also be designed with a small gap between contiguous edges of the spiral flexible member 101 even when the flexing member 101 is in the collapsed, generally flat state, to provide additional washing when the valve 100 is in an off state. Furthermore, a small hole 110 can be provided at generally the center of the valve 100 to aid in washing of the downstream side of the valve 100 as well as an additional leakage pathway for reverse blood flow.

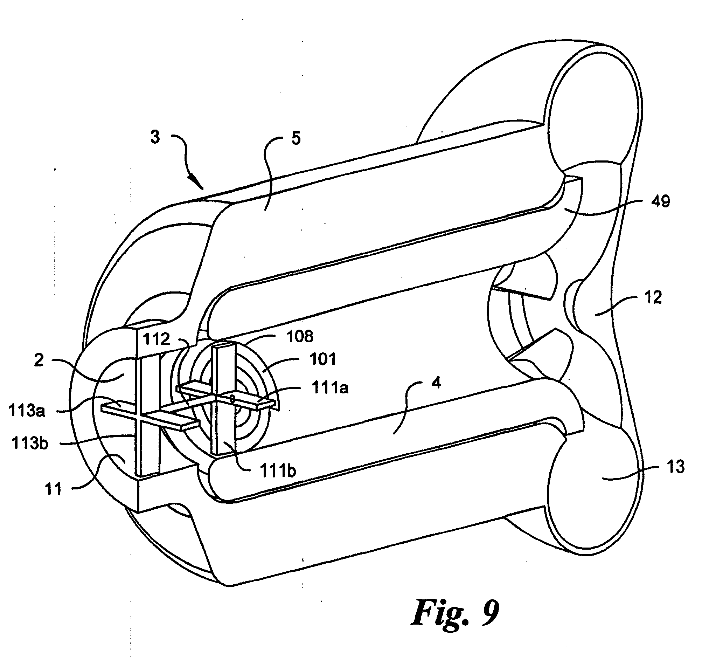

The valve 100 can have a support structure as depicted in Figure 9, wherein a pair of support struts 111a, 111b provide structural support to the largest diameter of the valve 100. The fixed end 102 of the valve 100 can be attached to the support struts 111a, 111b. The support struts 111a, 111b can be joined at the centers and have a central support member 112 extending away from the supports struts 111a, 111b. The central support member 112 can be mounted, in turn, to a set of cross members 113a, 113b which can be attached to the stator 4 near the inlet 11 of the blood pump 3, as shown in previous embodiments.

The direction of the spiral, e.g., clockwise or counter-clockwise, can be used to manipulate the blood flow as it passes through the flexible member 101. For example, the direction of the spiral can be in the same direction as the rotation of the rotor 4, or may be in the opposite direction. In this way, the behavior of the flow passing through the valve 100 can be manipulated to produce desirable flow effects. For instance, it may be desirable to have additional fluid swirling for blood entering the impeller 4a, in which case the flexible member 101 can spiral in the same rotational direction as the rotation of the rotor 4. Conversely, a flexible member 101 that spirals in a direction opposite the rotation of the rotor 4 will tend to decrease the swirling of the blood entering the impeller 4a. Coupled with the position of the valve 100 along the axis of rotation 6 of the rotor 4, i.e., close to or distant from the impeller 4a, an even more pronounced effect can be created for manipulation of blood flow entering the impeller 4a.

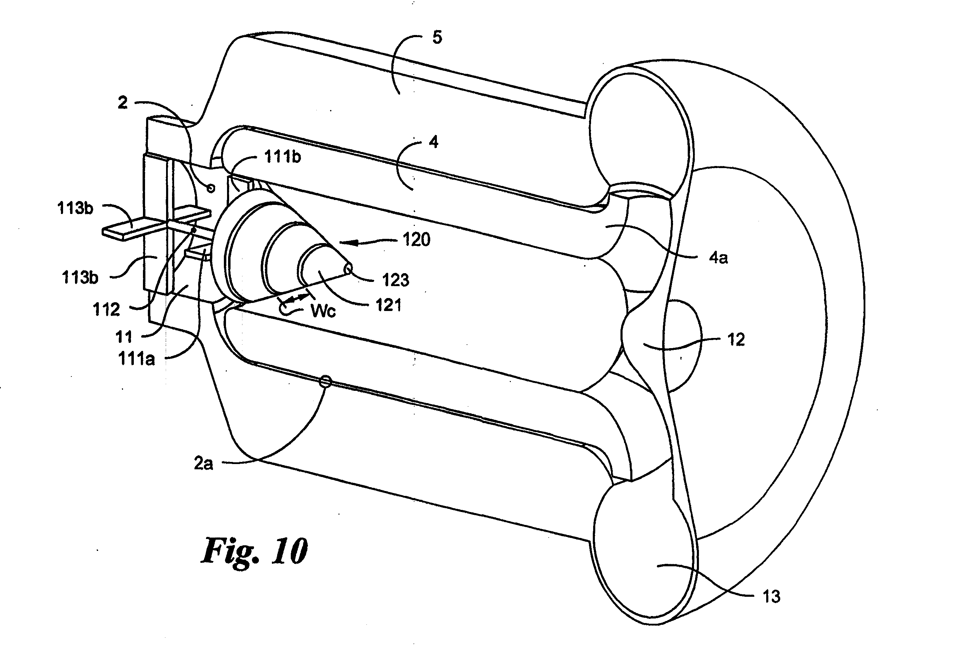

Another embodiment of a spiral valve 120 is depicted in Figure 10, wherein a continuous flexing member 121 is present within the central bore 2 of the blood pump 3. Like the previous embodiment, the flexing member 121 can have a spiral shape which, when collapsed, can substantially block the back-flow of blood. However, instead of being generally flat in the collapsed state, the flexing member 121 can instead form a generally conical shaped valve body. Like the generally flat spiral valve 100, when the blood pressure exceeds a predetermined level, the center portion of the flexible member 121 is designed to translate along the axis of rotation 6 of the rotor 4 in the direction of the blood flow, such that space forms between the edges of adjacent spirals to minimize impedance to blood flow. This space allows for the flow of blood through the conical body of the valve 120 and provides washing to the valve surface. Unlike the generally flat flexible member 101, the conical shaped flexible member 121 can provide better washing of the downstream side of the conical valve 120, since the width "W_{c}" of the flexible member 121 lies substantially parallel to the blood flow trajectory, rather than perpendicular to it as in the previous embodiment. Also a hole 123 at or near the center of the conical valve 120 can be provided similarly to the hole 110 in the flat spiral valve 100.

During a blood pump 3 off period, the conical valve 120 can preferably be washed in a manner similar to the previous embodiment. Other features of this embodiment can be likewise similar to the previous embodiment, including: placement within the blood pump 3 central bore 2, structural characteristics, materials, support structures, and the manner used to affect downstream flow.

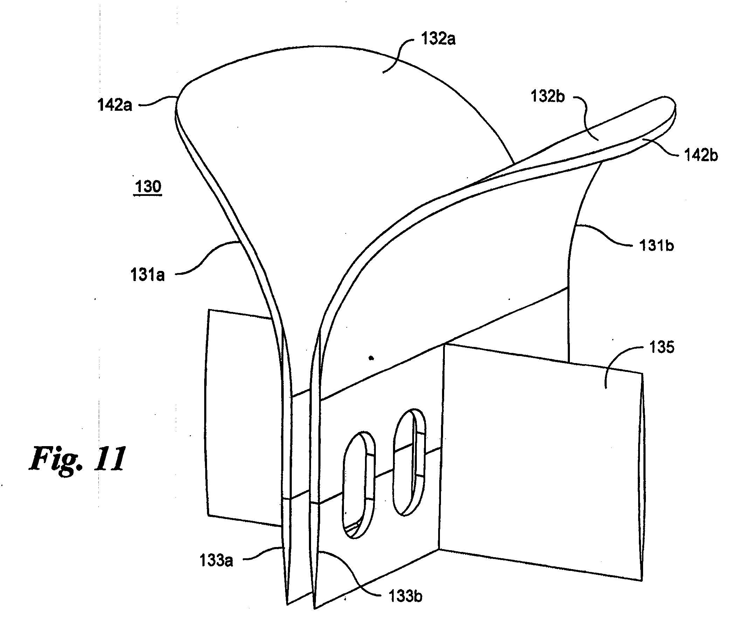

Another embodiment of the invention is depicted in Figure 11, wherein the valve member 130 has a pair of flexing members 131a, 131b which can be positioned in the central bore 2 of the blood pump 3. The flexing members 131a, 131b generally behave like the leaflets 52a-52d in the valve member 50 described previously. Changes in blood pressure cause the valve 130 to move from a closed state to an open state, and vice versa. In a closed state, as shown in Figure 11, the flexing members 131a, 131b can be flexed outward with respect to the axis of rotation 6 of the rotor 4. In an open state, the flexing members 131a, 131b can be generally parallel to the axis of rotation 6 of the rotor 4, such that a minimum profile is presented to the blood flow. In this way, the flexing members 131a, 131b can create a minimal pressure drop over the length of the valve 130.

The flexing members 131a, 131b can have upper portions 132a, 132b which provide the flexing movement, and a lower portions 133a, 133b which generally do not flex. The lower portions 133a, 133b can be mounted to a cross member 135 which can be mounted to the stator 5 near the inlet 11 of the blood pump 3. The cross member 135 can serve to structurally fix the valve 130 within the central bore 2 of the blood pump 3, and can produce advantageous flow effects either while the pump 3 operates or when the pump 3 is off. For instance, if the cross member 135 is angled with respect to the axis of rotation 6 of the rotor 4, swirling may be induced to the blood flow. Conversely, if the cross member 135 is angled opposite to the rotational direction of the rotor 4, the cross member 135 may tend to eliminate the swirling of blood entering the impeller 4a. The overall length of the flexing members 131a, 131b can be varied, by varying the length of one or both of the upper 132a, 132b and lower 133a, 133b portions, depending on the needs of the device, to further affect the degree of swirling in the blood entering the impeller 4a. This feature is similar to that explained in previous embodiments of the invention.

The two flexing members 131a, 131b can lie in close proximity to each other, and particularly the lower portions 133a, 133b thereof, and can be spaced about 0.015 inches apart. The amount of spacing can be determined so as to provide a pathway for blood to wash the surfaces of the flexing members 131a, 131b, and must be appropriately determined for when the flexing members 131a, 131b are open and when they are closed. In both instances, the spacing between the flexing members 131a, 131b can be generally constant along the length of the lower portions 133a, 133b, and can be large enough to provide adequate washing to prevent blood stagnation and clotting. Although generally parallel, i.e., generally constant spacing along the length of the fixed lower portions 133a, 133b, it should be understood that there could also be an angle therebetween.

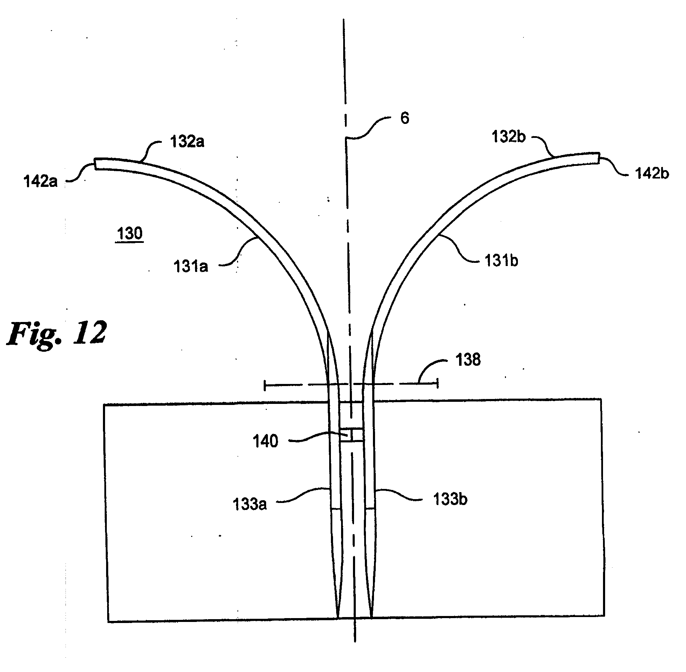

The valve 130 can be designed such that flexing occurs beyond the boundary 138 shown in Figure 12. The location of the boundary 138 can be defined by a support piece 140 positioned between the flexing members 130. The support piece 140, which may also be multiple support pieces, can have various shapes, sizes, or locations, but can be a fixed, generally rigid structure during valve 130 operation. The support piece 140 can be utilized to help define which portions of the flexing members 131a, 131b actually flex. This can be important due to the unknown load the valve 130 will operate under during normal conditions. For instance, although the magnitude of the pressure across the valve 130 for worst-case operation may be approximately determined, the actual flexural duty cycle imposed on the flexing members 131a, 131b can vary since every patient is different and will have different levels of physical activity. Flexure of the portion below the boundary 138 is not desirable due to the likelihood that the members 131a, 131b may touch and, with repeated contact, incur fatigue failure.

Thickness, material type , and shape can generally govern the flexural behavior of the flexing members 131a, 131b. Preferably, the flexing members 131a, 131b can have the spread, unloaded shape depicted in Figures 11 and 12. This position represents a closed state of the valve 130, such that, during pump 3 operation, the pressure gradient across the flexing members 131a, 131b bend the upper flexing portions 132a, 132b to a position more parallel to the axis of rotation 6 of the rotor 4. The energy stored due to the deflection is released when the pump 3 is not operational, such that the upper portions 132a, 132b spring back to the spread, or closed position.

In the closed position, the outer edges of the upper portions 132a, 132b preferably touch the wall 45 of the central bore 2 of the blood pump 3 at defined locations. Full contact may not be desirable, however, as blood flow across the outer edges of the flexing members 131a, 131b can provide the desired washing. In the open position, the flexing upper portions 132a, 132b are extended mostly parallel to the axis of rotation 6 of the rotor 4. This position allows the flexing members 130 to occupy a minimal amount of the central bore 2 cross-section, and consequently induce a minimal increase in pressure drop through the central bore 2. Designs that are too large may restrict the flow entering the impeller 4 too much, reducing the efficiency of the blood pump 3.

Each flexing member 131a, 131b can be made of Nitinol, and can have a thickness of about 0.002 inches. If needed, the thickness of the upper portions 131a, 131b in the flexing region may have a variable thickness to further control their behavior in response to pressure. Various features such as grooves, notches and channels of the peripheral edges 142a, 142b of the upper portions 132a, 132b may be added to improve valve washing.

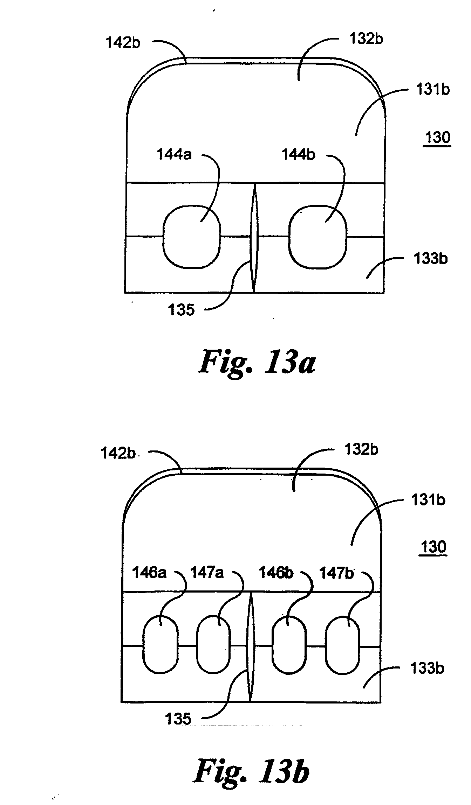

The projected area of each flexing member 131a, 131b may take the form shown in Figures 13a-13b. In these configurations, each flexing member 131a, 131b can have a hole or multiple holes 146a, 146b, 147a, 147b, through the thickness of each of the fixed lower portions 133a, 133b. The presence of such holes 144a, 144b can provide added pathways for blood to enter the tight space between the fixed lower portions 133a, 133b of the flexing members 131a, 131b. Although not required, it can be advantageous to have a different number of holes on flexing member 131a versus flexing member 131b. In addition, the shape of the holes 144a, 144b, 146a, 146b, 147a, 147b can also vary. Both the number of holes and the shape of the holes can be used to induce washing of the adjacent surfaces of flexing members 131a and 131b.

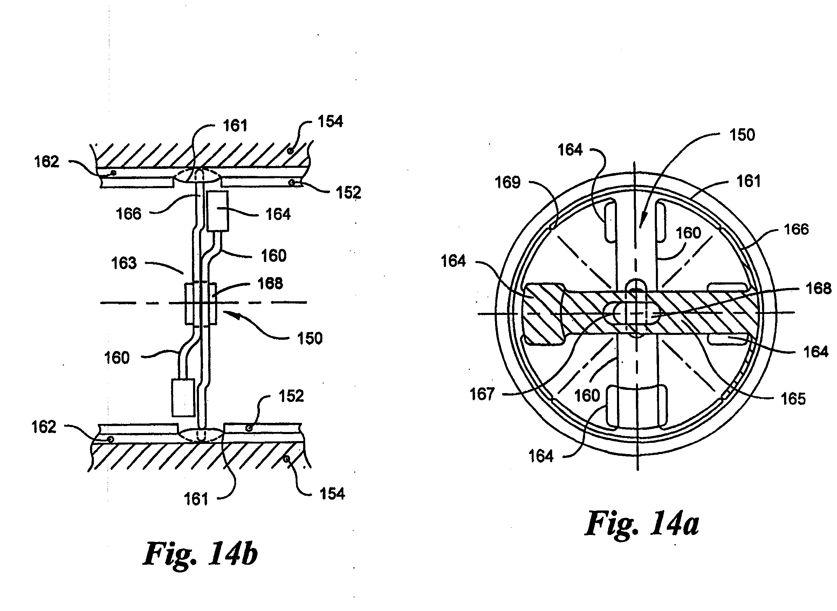

To address the control of flowrate through an annular secondary gap of a blood pump, for example, as illustrated in Figures 1, 3a-3b, 5a-5b, 7 and 9-10, which can also be similar to a blood pump as described in United States Patent No. 5,928,131, a circumferential valve may be employed. Such a circumferential valve may also be employed for a blood pump with only a single annular blood pathway. Different embodiments of circumferential valves are illustrated in Figures 14 through 20. Generally, such a circumferential valve can be open during normal operation of the blood pump, such that flow is unobstructed through the annular gap, or pathway, during normal blood pump operation. The switching of the valve state, open or mostly closed, can be made to occur responsive to centrifugal force created by rotation of the blood pump impeller, or can be controlled actively, such as electrically responsive to sensed rotational speed of the impeller. Active control can be accomplished, for example, using Nitinol as the actuating element.

Basically, such a circumferential valve can comprise an actuating mechanism covered by a polymeric membrane, wherein a portion of the polymeric membrane communicates with the annular gap/pathway. The actuating mechanism can move a portion of the polymeric membrane into the annular gap to provide the obstruction needed to reduce back-flow during periods when the blood pump is off, or when rotation of the impeller drops below a predetermined speed. The actuating mechanism can be associated with either the rotor or the stator of the blood pump.

In the embodiments shown in Figures 14a and 14b, such a circumferential valve can comprise a pusher member, or multiple pusher members, carried by the rotor. The pusher member can be attached to the rotor with one end in contact with the polymeric membrane where the membrane communicates with the annular gap. The pusher member can be designed to push the membrane into the annular gap, thereby mostly obstructing the annular gap when the rotor is stationary, or rotating at low speeds. At normal rotational speeds, the rotor generates centrifugal force sufficient to cause the pusher member to move in a direction which retracts, or permits retraction of, the membrane from the annular gap. The valve can be designed to remain open for rotor/impeller speeds above, for example, about 1,000 RPM. At an impeller velocity of roughly 0 RPM up to about 1,000 RPM, the valve can preferably be fully employed, i.e., the membrane is pushed into the annular gap, thereby producing partial occlusion of the annular space between the rotor and the stator. This can prevent a substantial loss of pressurized aortic blood that could otherwise flow backward through the secondary gap into the left ventricle when the impeller is rotating at slower speeds.

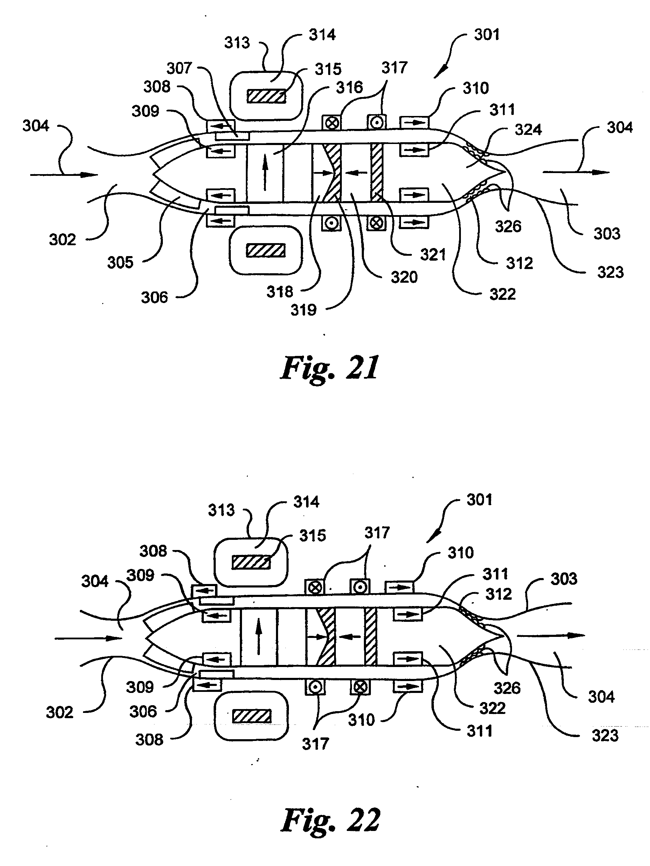

The actuating mechanism 150 can be located in a rotor portion of a blood pump 3. This type of circumferential valve can be more suitable for a single flow path blood pump, such as shown in Figures 21 and 22, since the actuating mechanism can be housed inside the rotor portion 152 of the blood pump. As such, the actuating mechanism 150 would not be positioned in a blood flow path, such as the main blood flow path, i.e., the central bore 2, for example, as shown in Figures 3a and 3b. The actuating mechanism 150 can have multiple sliding members 160, four shown, which change position depending on rotor speed. A polymeric membrane 161 can encircle the sliding members 160 such that during blood pump 3 operation, the annular gap 162 between the rotor 152 and the housing 154 is generally uniform across the back-flow valve 163.

Each sliding member 160 can have a weighted end 164, a flat slotted member 165, and a pusher-bar 166. The center portion of each sliding member 160 can have a slot 167 that is positioned for a sliding pin 168. The pin 168 can hold the center of all four sliding members 160. At normal operational speeds, the rotor 152 rotation can induce a centrifugal force sufficient to cause the weighted ends 164 of the sliding members 160 to move outward radially, away from the axis of rotation of the rotor 152. In this position, the sliding members 160 can be in a fully retracted state, causing no general obstruction of the annular gap 162 between the stator 154 and rotor 152. Below normal operational speeds, the sliding members 160 can retract to a position that forces the pusher-bar 166 end of the sliding member 160 into the annular gap 162. The retraction of the sliding members 160 can be accomplished, for example, through preloading of the polymeric membrane 161 that covers the sliding member 160 region. Also, the retraction can also be accomplished, for example, through preloaded compression springs that force the pusher-bar 166 of the sliding members 160 out of the annular gap 162 between the rotor 152 and stator 154. The pusher-bars 166 can have a rounded outer surface with rounded ends 169 that can safely push against the polymeric membrane 161 to the extent needed for flow reduction, without causing excessive stresses in the polymeric membrane 161.

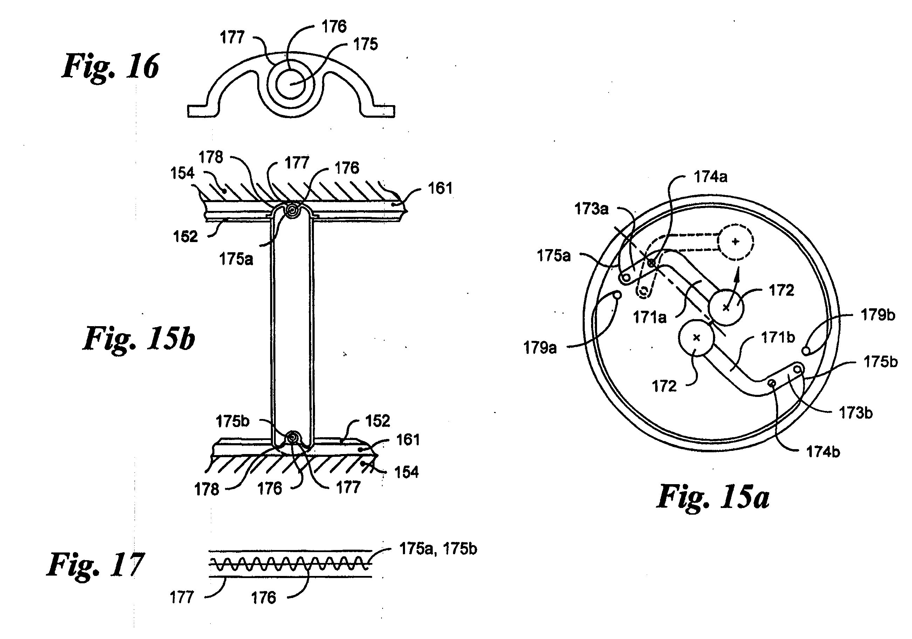

Another embodiment a circumferential valve 170 is depicted in Figures 15a through 17 shown having two pivoting arms 171a, 171b that can also be located within the rotor 152. Each pivoting arm 170a, 170b can have a weighted end 173a, 173b and an opposite end 172a, 172b that can be connected to a cable 175a, 175b. The weighted end 172a, 172b can preferably be farther removed from the pivot point 174a, 174b of the arm 171a, 171b, whereas the cable end 173a, 173b can be substantially closer. The cable 175a, 175b attached to each arm 171a, 171b can extend through a low friction coil 176, which in turn can be contained within a channel 177, as shown in Figures 16 and 17. The channel 176, which can be a polyurethane material, can also be an integral portion of the polyurethane membrane 178 that runs circumferentially around the rotor 152. In the relaxed state during periods when the pump is not powered, the polyurethane membrane 178 can be in a radial position with respect to the annular gap 162, i.e., blood pathway, such that partial occlusion of the annular gap 162 can be accomplished to an extent sufficient to prevent a substantial back-flow of pressurized blood from the patient's heart. As with the previous embodiment, the actuating mechanism 170 can retract during rotor rotational speeds above approximately 1000 RPM, such that the blood pathway 162 is generally uniformly annular with minimal obstruction due to the polyurethane membrane 178. The pivoting action of the arms 171a, 171b about the center of rotation 174a, 174b (shown in dashed lines in Figure 15a) can be caused by the centrifugal force, which moves the weighted-ends 172a, 172b of the arms 171a, 171b outward when the rotor 152 rotates at speeds above 1000 RPM. The cable-end 173a, 173b of each arm 171a, 171b pulls a proportional amount of cable 175a, 175b through the polyurethane channel 177. The opposite end of the cable 175a, 175b can be fastened to a pin 179a, 179b that is fixed with respect to the rotor 152. The shortening of the cable 175a, 175b within the polyurethane channel 177 effectively provides circumferential shortening of the polyurethane channel 177. To accommodate this shortening, the polyurethane membrane 178 can snap through to a position, shown by dashed line at the bottom of Figure 15b, within the envelope of the rotor 152, thus generally eliminating any obstruction of the blood flow pathway 162. The low friction coil 176 situated between the polyurethane channel 177 and the cable 175a, 175b can provide a surface for the cable 175a, 175b to rub against, thus preventing abrasion of the polyurethane channel 177 as the cable 175a, 175b is pulled through its length.

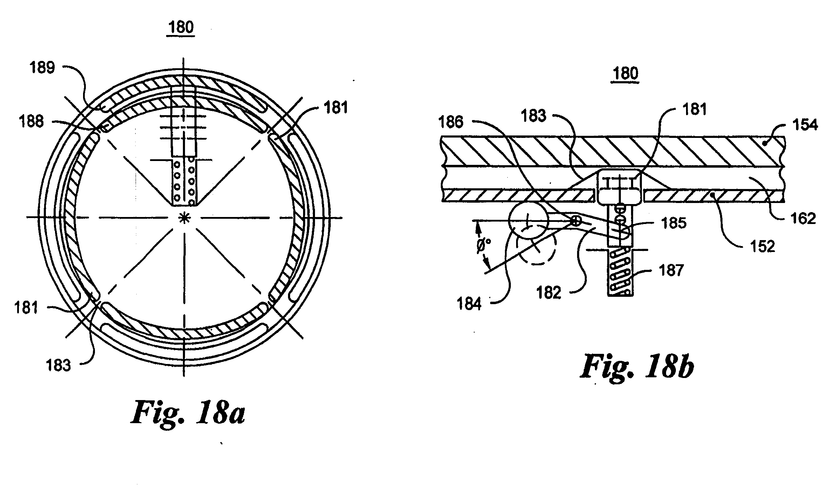

Another similar embodiment is depicted in Figures 18a and 18b, wherein a pusher-bar 181 and a pivoting arm 182 can be combined into a speed regulated valve actuating mechanism 180. Although, for convenience and to simply the drawing only one pivot arm 182 is shown, multiple, for example, four pivot arms can be circumferentially positioned around the interior of the rotor 152. Each pivot arm 182 can have a pusher bar 181 that rests against a circumferential polymeric membrane 183, and can pivot about an end 184 of the pivot arm 182. The opposite end of the pivot arm 182 can be a weighted end 185. Between the pusher bar 181 and weighted end 184 of the pivot arm 182 can be a rotational center 186 about which the pivot arm 182 rotates. The pivot arm 182 can be designed to rotate through a small angle, Ø, which can be about 30°. A spring 187 can be positioned below each pusher bar 181 such that the pusher bar 181 is biased against the polymeric membrane 183, causing the membrane 183 to invade the annular blood pathway 162 to an extent sufficient to minimize back-flow, as explained in previous embodiments. When the rotor 152 rotates at speeds above approximately 1000 RPM, centrifugal force can cause the weighted ends 184 to move outward radially, which can in turn can cause the pivot arm 182 to rotate such that the pusher bar 181 moves inward radially. Consequently, the annular blood space 162 becomes generally unobstructed when the rotor 152 speed exceeds about 1000 RPM. When the rotor speed drops below about 1000 RPM, the spring 187 can push the pusher bar 181 from its inner position 188 back to the outer position 189. Likewise, the membrane 183 can be moved from the inner position 188 to the outer position 189.

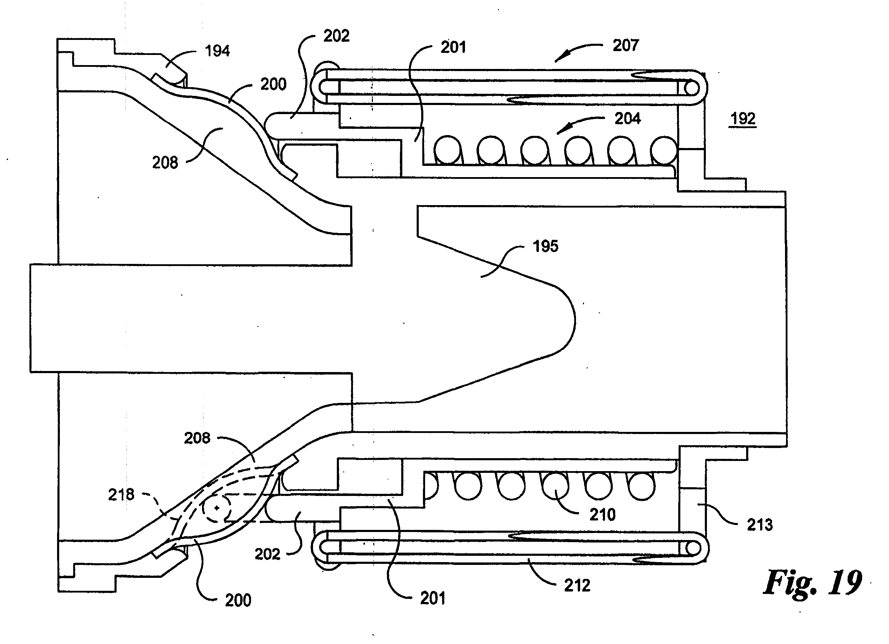

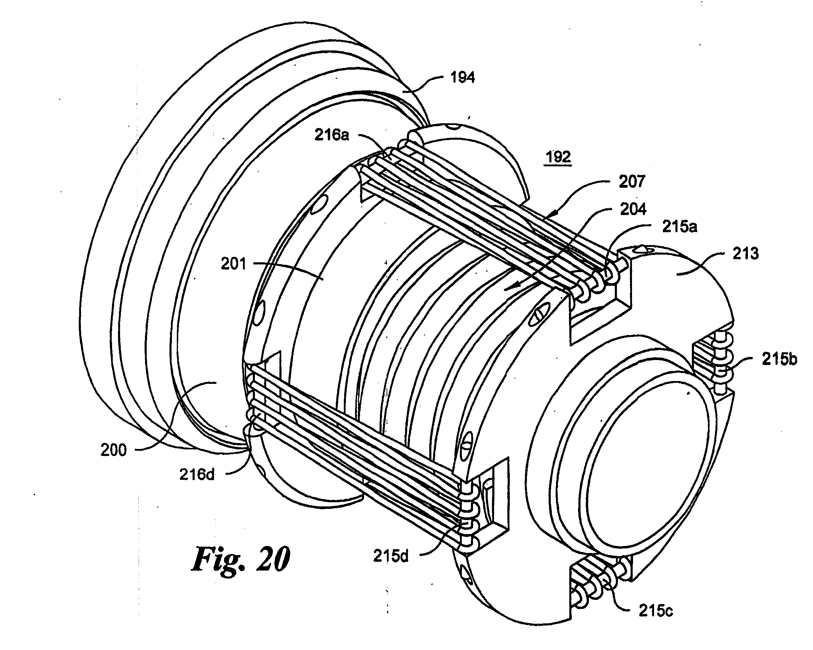

Referring now to Figures 19 and 20, another embodiment of an actuating mechanism 192 can be associated with a stator portion 194 of a blood pump. The actuating mechanism 192 generally comprises a membrane 200 movable by a pusher member 201. A first control member 204 and a second control member 207 can be provided to control the position of the pusher member 201. For example, the first control member 204 could be employed to bias the pusher member 201 to hold the membrane 200, or a portion thereof, in the annular gap 208. The second control member 207 could be selectively activated to overcome the bias of the first control member 204 and permit the membrane 200 to withdraw from the annular gap 208. The polymeric membrane 200 can form part of the stator wall 194, in contact with the annular gap 208 between the rotor 195 and stator 194 . The pusher member 201 can be positioned external to the membrane 200, and can have an annular element with a circumferential portion 202 which is pushed against the polymeric membrane 200. The pusher member 201, under the influence of the first control member 204, can bias the membrane 200, or a portion thereof, into the annular gap 208 between the rotor 195 and stator 194 to create an obstruction which substantially, but not entirely, blocks reverse to back-flow. The first control member 204 can cause the pusher member 201 to normally hold the membrane 200 in the annular gap between the rotor 195 and stator 194 when the rotor 195 is stopped or operating below a certain rotational speed. The first control member 204 can, for example, be a resiliently compressible member, such as a compression spring 210, and can be pre-loaded between the pusher member 201 and a ground element 213. The ground element 213 can have an annular shape, and can be rigidly attached to the stator 194. The ground element 213 and the annular pusher member 201 can each have four stationary pins 215a-215d and 216a-216d, respectively, located about an outer periphery thereof. The pins 215a-215d can be spaced equally and can be aligned with each other such that each pin 215a-215d on the pusher member 201 is aligned with a corresponding pin 216a-216d on the ground element 213. The second control member 207 can be, for example, Nitinol wire 212, which can be wound around the pins 215a-215d of the pusher element 201 and the corresponding pins 216a-216d on the ground element 213, such as in the manner depicted in Figure 20. In the pump off state, the first control member 204 can hold the pusher member 201 against the polymeric membrane 200, such that the membrane, or a portion thereof, is pushed into the annular gap 208 between the rotor 195 and the stator 194, as shown by dashed lines 218 in Figure 19. The positioning of the pusher member 201 and the polymeric membrane can 200 serve to minimize the level of back-flow through the annular blood gap 208 to reduce the leakage through the blood pump when the rotor 195 is stopped, or rotating below a certain speed. The second control member 207 can be selectively activated, such as responsive to sensed rotor 195 speed, to overcome the biasing force exerted by the first control member 204 and permit the membrane 200 to be withdrawn from the annular gap 208. For example, current can be applied to the Nitinol wire 212, causing the wire to shorten, thus compressing the compression spring 210 and decreasing the distance between the ground element 213 and the annular element 201. This moves the pusher member 201 axially away from the polymeric membrane 200, allowing the membrane 200 to withdraw from of the annular blood gap 208. In sum, the membrane 200 substantially occludes the annular space 208 when no current is applied to the Nitinol wire 212, and is substantially removed from the annular space 208 when current is applied to the Nitinol wire 212. When current is discontinued to the Nitinol wire 212, the compression spring 210 can provide the necessary force to return the pusher member 201 to its axial rest position wherein the membrane 200 is pushed into the annular gap 208.

Figures 21 and 22 illustrate an embodiment of a single gap blood pump 301 having a stator 323 in which is disposed for rotation a rotor 322, wherein the rotor is axially movable within the stator 323 to substantially, but not entirely, block the blood flow path. The rotor 322 can be magnetically supported within the stator 323 for rotation therein to pump blood through the blood pump 301 along a blood flow path indicated by arrows 304, from a pump inlet 302 through a pump outlet 303. In the particular embodiment shown, the blood pump 301 is an axial flow design. The rotor 322 can be magnetically suspended within the stator 323 by, for example, stator magnet bearing portions 308 and 310 in cooperation with respective, generally adjacent, rotor magnet bearing portions 309 and 311. Permanent magnets can be used for some, or all, of the stator 308, 310 and rotor 309, 311 magnet bearing portions. As shown, rotor bearing magnet portions 309 and 311 are inner magnet rings carried by the rotor 322, whereas stator bearing magnet portions 308 and 310 are outer magnet rings carried on the stator 323. The permanent magnet bearing portions 308-311 can provide a positive centering stiffness, which centers the rotor radially within the housing. However, the magnet bearing portions 308-311 can have a negative stiffness in the axial direction such that the rotor 322 may be unstable axially within the stator 323. Therefore, a linear motor can be used to axially support the rotor 322 within the stator. As shown, the linear motor shown can be comprised of a magnet assembly 318-321 in cooperation with coils 317. The linear motor can be servo controlled responsive to an axial position sensor (not shown) which may be an eddy-current type sensor, of a type which is well known in the art. Alternatively, other types of position sensors known to those of skill in the art can also be utilized. The magnet assembly 318-321 can include iron pole pieces 319 and 321 and permanent magnets 318 and 320. Pole piece 319 is can be a North pole and pole piece 321 can be a South pole. The magnet assembly 318-321 can interact with current in coils 317 to produce axial forces. The positive current direction in the coils is depicted with the standard arrow tip and arrow tail notation. Note that the currents in the coils are in opposite directions.

As shown by the arrows 304, blood flows into the pump via the inlet 302 and exits via outlet 303. The rotor 322 can include impeller blades 305 and stator blades 307 which propel and control the flow of blood through the blood pump 301. The impeller blades 305 and stator blades 307 can both be helical. A DC brushless motor can be utilized, which can include a stator motor portion 313 and a rotor motor portion 316, which cooperate to rotate the rotor 322, and thus the impeller blades 305. The rotor motor portion 316 can be a permanent magnet. The stator motor portion 313 can include an annular laminated iron ring 315 and six toroidally wound coils 314, which form a 2 pole, 3-phase motor. Alternatively, it is to be understood that other types or configurations of motors can be devised by those of skill in the art.

Referring particularly to Figure 22, an integral back-flow limiting valve can be provided such that if the blood pump 301 were to lose power, or be purposely powered down, reverse blood flow through the blood pump 301 would be restricted, but not entirely blocked. The back-flow limiting valve can be integrally formed by providing for axial movement of the rotor 322 within the stator 323, and by specially designing the pump outlet 303, or the stator 323 at the pump outlet 303, and an aft portion 324 of the rotor 323 adjacent the pump outlet 303. The pump outlet 303 and rotor aft portion 324 can be configured such that axial movement of the rotor 323 brings the aft portion 324 into a nearly, but not completely, sealing engagement with the pump outlet 303. In this manner, back-flow is not completely eliminated, but instead a small retrograde blood flow through the blood pump 301 is permitted which washes the blood flow path surfaces and helps prevent clot formation.

To accomplish axial movement of the rotor 322, such as during power or other failures, the coils 317 can move the rotor 322 aft, toward the outlet 303, thereby restricting reverse, or regurgitant, flow of blood by closing the gap 312 between the rotor aft portion 324 and the stator 323, i.e., pump outlet 303, as shown in Figure 22. A sufficient gap 306 can be provided between the rotor blades 305 and the stator blades 307 to allow axial movement of the rotor 322 in the aft direction without interference between the blades 305, 307. The stator blades 307 can be appropriately configured, for example, having a cylindrical inner tip geometry, to permit the rotor 322 to move towards the pump outlet 303 without interference between the stator blades 305 and the rotor 322 or rotor blades 307. Once moved aft, the rotor 322 will tend to remain in the displaced position due to the unstable character of the permanent magnet bearing portions 308-311. It can be necessary that the coils 317 move the rotor 322 aft during failure, since a forward position of the rotor 322 is possible if there is no power to the blood pump 301. It is also to be understood that there are many possible magnetic suspension and drive configurations which can be utilized consistent with this the invention, wherein the rotor 322 is moved axially within the stator 323 to restrict retrograde blood flow in the event of cessation of power to the blood pump 301.

In order to allow a limited reverse flow through the blood pump 301 after the rotor 322 has been moved aft, the rotor 322, stator 323, or both, may be designed so as not to be perfectly axi-symmetric. For example, small "bumps" or "dips" 326 in the aft portion 324 of the rotor and/or the inner surface of the stator 323 at the pump outlet 303, can be provided to prevent a complete seal of the outlet 303 when the rotor 322 has been axially moved. This configuration creates small gaps between the aft portion 324 of the rotor 322 and the pump outlet 303, or stator 323, which allow for a small amount of retrograde flow through the outlet 303 after the rotor 322 has been moved aft. The degree of reverse blood flow enabled can be controlled by the design of the mating portions of the rotor 322 and pump outlet 303, or stator 323, e.g., by controlling the size and/or shape of the bumps/dips 326. The small amount of back flow washes the blood flow path 304 and helps prevent clot formation.

In the various embodiments described, a back-flow-limiting valve member can be included within a blood pump to numerous advantages. The advantages can include increasing safety by preventing excessive reverse flow through the blood pump during periods of non operation, increasing therapeutic flexibility by enabling the blood pump to be turned off when not needed for circulatory support, increasing the time between charges of internal and external batteries due to the intermittent blood pump operation, and increasing the usable life of the blood pump as a result of said intermittent operation.

Additionally, although certain embodiments of the invention have been described in detail, it will be appreciated by those skilled in the art that various modifications to those details could be developed in light of the overall teaching of the disclosure. Accordingly, the particular embodiments disclosed herein are intended to be illustrative only, and not limiting to the scope of the invention, which should be awarded the full breadth of the following claims and any and all embodiments thereof.

## Claims

1. A blood pump comprising an inlet, an outlet, a rotor cooperating with a stator for pumping blood through at least one blood flow path from said inlet to said outlet, said rotor movable axially with respect to said stator between a pumping position and a non-pumping position, a portion of said rotor configured to substantially but not entirely restrict a reverse flow of blood from said outlet to said inlet when said rotor is moved to said non-pumping position such that a limited reverse blood flow is permitted.

2. A blood pump comprising an inlet, an outlet, a rotor cooperating with a stator for pumping blood through at least one blood flow path from said inlet to said outlet, and a back flow check valve member substantially but not entirely restricting a reverse flow of blood from said outlet to said inlet such that a limited reverse flow is permitted responsive to a predetermined reduction in rotation speed of said rotor.

3. The blood pump of claim 2 wherein said back flow check valve member further comprises a balloon member centrally positioned in said at least one blood flow path, said balloon member having a fixed end attached to said stator, said balloon member inflatable to substantially but not entirely block said at least one flow path to permit said limited reverse flow and contractible to present a minimal impedance to forward blood flow.

4. The blood pump of claim 3 further comprising said fixed end of said balloon member attached to said stator near said outlet.

5. The blood pump of claim 3 further comprising said fixed end of said balloon member attached to at least one cross member and said at least one cross member attached to said stator near said inlet.

6. The blood pump of claim 5 further comprising said at least one cross member having a wide dimension and a thin dimension and said at least one cross member positioned generally parallel to and at least partially across said at least one blood flow path such that said thin dimension presents minimal impedance to blood flow.

7. The blood pump of any one of claims 3-6 further comprising:
a. said balloon member having an elliptical shape with a long axis generally coaxial with an axis of rotation of said rotor; and
b. said balloon member having plurality of curved lobe portions, said plurality of curved lobe portions defining a maximum diameter at a midpoint of said balloon member, said maximum diameter substantially but not entirely equal to a diameter of said blood flow path.

8. The blood pump of claim 7 further comprising a support frame disposed within said balloon member, said support frame having at least one lobe support member which supports at least one of said plurality of curved lobe portions of said balloon member, said support frame having at least one passageway therethrough, one end of said at least one passageway connectable to a source of pressure and another end of said passageway communicating within said balloon member to at least one of inflate and deflate said balloon member.

9. The blood pump of any one of claims 2-8 wherein said back flow check valve member further comprises a disk member centrally positioned in said at least one blood flow path, said disk member pivotable to a first position wherein a face of said disk member substantially but not entirely blocks said at least one blood flow path to permit said limited reverse flow between an outer periphery of said disk and a bore of said stator which defines said at least one blood flow path, and said disk member pivotable to a second position wherein a thickness of said disk member is substantially aimed along said at least one blood flow path so as to present minimal obstruction of forward blood flow.

10. The blood pump of claim 9 further comprising a strut having a first end pivotably connected to said disk member and a second end connected to said stator near said outlet.

11. The blood pump of claim 10 further comprising at least one cross member, said second end of said strut attached to said at least one cross member, and said at least one cross member attached to said stator near said inlet.

12. The blood pump of claim 11 further comprising said at least one cross member having a wide dimension and a thin dimension and said at least one cross member positioned generally parallel to and at least partially across said at least one blood flow path such that said thin dimension presents minimal impedance to blood flow.

13. The blood pump of claim 11 or claim 12 further comprising said at least one cross member positioned at an angle to said at least one blood flow path such that characteristics of blood flow across said at least one cross member are affected.

14. The blood pump of any one of claims 9-13 further comprising said outer periphery of said disk member configured to enhance said limited reverse blood flow when said disk member is in said first position.

15. The blood pump of any one of claims 9-14 further comprising a hole thru said face of said disk member.

16. The blood pump of any one of claims 9-15 further comprising said disk member biased in said first position and said disk member moving to said second position when sufficient forward flow is present to pivot said disk to said second position.

17. The blood pump of any one of claims 2-16 wherein said back flow check valve member further comprises a flapper valve centrally positioned in said at least one blood flow path, said flapper valve having a plurality of leaflets movable between a first position wherein faces of said plurality of leaflets substantially but not entirely block said at least one blood flow path to permit said limited reverse flow between an outer periphery formed by said plurality of leaflets and a bore in said stator which defines said at least one blood flow path, and a second position wherein a thickness of each of said plurality of leaflets is substantially aimed along said at least one blood flow path so as to present a minimum obstruction to forward blood flow.

18. The blood pump of claim 17 further comprising at least one cross member and said plurality of leaflets supported by said at least one cross member.

19. The blood pump of claim 17 wherein said plurality of leaflets is four leaflets and further comprising a pair of cross members and a pair of said four leaflets supported by each of said pair of cross members.

20. The blood pump of claim 18 or claim 19 further comprising said at least one cross member attached to said stator near said inlet.

21. The blood pump of claim 18 or claim 19 further comprising a strut having a first end attached to said at least one cross member and a second end attached to said stator near said outlet.

22. The blood pump of claim 21 further comprising at least one additional cross member, said second end of said strut attached to said at least one additional cross member, and said at least one additional cross member attached to said stator near said inlet.

23. The blood pump of any one of claims 18-22 further comprising said at least one cross member having a wide dimension and a thin dimension and said at least one cross member positioned generally parallel to and at least partially across said at least one blood flow path such that said thin dimension presents minimal impedance to blood flow.

24. The blood pump of any one of claims 18-23 further comprising said at least one cross member positioned at an angle to said at least one blood flow path such that characteristics of blood flow across said at least one cross member are affected.

25. The blood pump of any one of claims 18-24 further comprising a generally cylindrical support member attached to at least one of said at least one cross member and at least one of said plurality of leaflets, and said generally cylindrical support member having a diameter substantially but not entirely equal to a diameter of said blood flow path such that said limited reverse blood flow occurs around an outer periphery of said generally cylindrical support member.

26. The blood pump of any one of claims 17-25 further comprising said outer periphery formed by said plurality of leaflets configured to one of increase and decrease said limited reverse blood flow when said disk member is in said first position.

27. The blood pump of any one of claims 17-26 wherein said plurality of leaflets are comprised of a flexible material such that movement between said first and second positions is accomplished via bending of said flexible material.

28. The blood pump of claim 27 wherein said flexible material comprises a material which contracts and expands responsive to electrical stimulation.

29. The blood pump of claim 27 or claim 28 wherein said flexible material further comprises Nitinol.

30. The blood pump of any one of claims 2-29 wherein said back flow check valve member further comprises a substantially planar spiral valve centrally positioned in said at least one blood flow path, said spiral valve being a continuous flexible member having one fixed end and one free end extending from said fixed end in a spiral manner in a common plane with said fixed end, said continuous flexible member movable between a first position wherein said free end is generally planar with said fixed end, and a second position wherein said free end is translated in a direction generally normal to said common plane such that a generally conical shape is formed, said spiral valve substantially but not entirely blocking said at least one flow path in said first position to permit said limited reverse flow, and said spiral valve presenting a minimum impedance to forward blood flow in said second position.

31. The blood pump of claim 30 further comprising at least one cross member and said free end attached to said at least one cross member.

32. The blood pump of claim 31 further comprising said at least one cross member attached to said stator near said inlet.

33. The blood pump of claim 31 or claim 32 further comprising said at least one cross member having a wide dimension and a thin dimension and said at least one cross member positioned generally parallel to and at least partially across said at least one blood flow path such that said thin dimension presents minimal impedance to blood flow.

34. The blood pump of any one of claims 31-33 further comprising said at least one cross member positioned at an angle to said at least one blood flow path such that characteristics of blood flow across said at least one cross member are affected.

35. The blood pump of claim 31 further comprising a strut having one end attached to said at least one cross member and a second end attached to said stator near said outlet.

36. The blood pump of claim 35 further comprising at least one additional cross member, said second end of said strut attached to said at least one additional cross member, and said at least one additional cross member attached to said stator near said inlet.

37. The blood pump of any one of claims 30-36 further comprising no gap between adjacent edges of said continuous flexible member.

38. The blood pump of any one of claims 30-36 further comprising a gap between adjacent edges of said continuous flexible member such that some reverse blood flow is permitted through said spiral valve via said gap when said free end is at said first position.

39. The blood pump of any one of claims 30-38 further comprising said continuous flexible member having a thickness which varies along a length thereof from said fixed end to said free end.

40. The blood pump of any one of claims 30-39 further comprising said continuous flexible member having a width which varies along a length thereof from said fixed end to said free end.

41. The blood pump of any one of claims 30-40 further comprising at least one hole in said continuous flexing member.

42. The blood pump of claim 41 further comprising said at least one hole located at said free end of said continuous flexing member near a center of said spiral valve member.

43. The blood pump of any one of claims 30-42 wherein said free end of said continuous flexing member extends outward from said fixed end in a spiral manner into a different plane from said common plane such that in said first position said spiral valve has a generally conical shape, and in said second position said free end is translated further away from said fixed end such that said spiral valve has an extended conical shape when said free end is at said second position.
